# FASCICULE DE BREVET EUROPEEN

(11) **EP 3 024 388 B1**
(45) Date de publication et mention de la délivrance du brevet: **06.09.2017**
(21) Numéro de dépôt: 14767122.6
(22) Date de dépôt: 24.07.2014
(51) Int. Cl.: A61B 5/053, A61B 5/145, A61B 5/00, G01N 33/487

(54) **DISPOSITIF D'EVALUATION DE LA PERTE HYDRIQUE D'UN INDIVIDU OU D'UN ANIMAL PAR SUDATION**
VORRICHTUNG ZUR BEURTEILUNG DES WASSERVERLUSTES EINER PERSON ODER EINES TIERES DURCH SCHWITZEN
DEVICE FOR ASSESSING THE WATER LOSS OF A PERSON OR OF AN ANIMAL THROUGH PERSPIRATION

(30) Priorité: 25.07.2013 FR 1357344
(43) Date de publication de la demande: 01.06.2016
(73) Titulaire: Commissariat à l'Energie Atomique et aux Energies Alternatives, 75015 Paris (FR)
(72) Inventeur: REVOL-CAVALIER, Frédéric, F-38180 Seyssins (FR); MARSIQUET, Cyril, F-16270 Roumazieres Laubert (FR)
(74) Mandataire: Nony
(86) Numéro de dépôt international: PCT/IB2014/063375
(87) Numéro de publication internationale: WO 2015/011668

(56) Documents cités:
- EP-A1- 2 682 745
- WO-A1-2013/114273
- FR-A1- 2 453 631
- FR-A1- 2 968 532
- JP-A- 2010 046 196

## Description

La présente invention concerne un dispositif destiné à évaluer la perte hydrique de la personne par sudation et adapté pour être porté par le sujet.

De nombreux travaux ont montré l'intérêt d'une bonne hydratation chez les individus, notamment lors d'activités physiques sportives ou chez les personnes fragiles comme les nourrissons ou les personnes âgées. La perte hydrique due à la transpiration ou le manque d'hydratation peut entraîner l'apparition de troubles physiologiques, par exemple, la perte de poids, des troubles de la concentration, une fatigue importante ou des vertiges. Pour les cas de déshydratation les plus sévères, des pertes de facultés intellectuelles ou de troubles physiologiques pouvant entraîner la mort de l'individu ou de l'animal peuvent également survenir.

La sudation est le principal moteur de la régulation thermique du corps humain et de certains animaux supérieurs. Lorsque la sudation devient insuffisante pour refroidir le corps, la température corporelle interne augmente. Pour éviter la déshydratation ou la surhydratation, une approche consiste à suivre la température interne du corps selon des techniques classiques de mesure de température du corps. Toutefois, les mesures externes de la température corporelle, par exemple axillaires, ne sont pas suffisamment précises et rapides pour un tel suivi. D'autre part, les mesures internes de la température corporelle, par exemple buccales, rectales ou auriculaires, ont un usage restreint, limité au laboratoire et au milieu médical, et sont peu adaptées à un usage en continu et en temps réel.

Une autre approche consiste à évaluer la perte hydrique par sudation, c'est-à-dire la quantité de sueur transpirée sur un temps donné. Cette évaluation est, classiquement, pratiquée par pesée sur une balance différentielle. L'individu est pesé à plusieurs reprises, tout au long d'un exercice physique ou lors du contrôle pour permettre de calculer le rapport entre la perte hydrique et le poids de l'individu. Ce rapport est caractéristique du taux de déshydratation de l'individu et, par conséquent, de son état d'hydratation. Cette solution est précise et sensible, mais ne demeure applicable qu'en laboratoire, et ne peut convenir à des applications nomades ou hors milieu médical.

Différents dispositifs portés par l'individu ont été proposés pour évaluer la perte hydrique d'un individu à partir de la mesure du débit de sueur sécrétée. Par exemple, le document JP 2010-046196 décrit un dispositif de mesure de sudation comportant un matériau absorbant muni d'un indicateur coloré changeant de couleur au contact de la sueur. Le dispositif intègre un film au contact de la peau, dans lequel est aménagée une ouverture afin d'exposer le matériau absorbant et permettre l'introduction et l'absorption de la sueur au sein du matériau absorbant. La quantité de sueur sécrétée est évaluée par visualisation de la progression de la sueur teintée par l'indicateur coloré le long du matériau absorbant.

On peut encore citer le dispositif porté, proposé dans le document FR 2 968 532 pour évaluer un taux d'excrétion d'un fluide corporel, par exemple la sueur, par un individu ou un animal, qui intègre un élément absorbant muni d'électrodes de mesure. Une enveloppe imperméable, disposée entre la source d'excrétion du fluide corporel et l'élément absorbant, est munie d'un orifice d'entrée exposant une partie de l'élément absorbant pour assurer le passage du fluide corporel à travers l'élément absorbant.

Dans ces deux types de dispositifs portés, la collecte et la mesure de la quantité de sueur collectée sont ainsi assurées par un matériau unique. Or, dans ces conditions, si ce matériau est un matériau apte à drainer la sueur, la sueur collectée va se répandre rapidement sur la totalité de la zone de mesure, ce qui est susceptible d'entraîner une erreur de mesure sur la quantité de sueur stockée par le dispositif. En revanche, si ce matériau est un matériau absorbant, une partie de la sueur va rester sur la zone de collecte, ce qui sera alors préjudiciable à la détection de faibles quantités de sueur. D'autres exemples des dispositifs connus sont les documents FR 2 968 532 A1, JP 2010 046196, FR 2 453 631 A1, EP 2 682 745 A1 et WO 2013/114273 A1. La présente invention vise précisément à perfectionner les dispositifs portés de détermination de la perte hydrique par sudation d'un individu, afin de permettre une évaluation fiable et précise de la quantité de sueur sécrétée par cet individu.

Plus particulièrement, la présente invention concerne, selon un premier de ses aspects, un dispositif d'évaluation de la perte hydrique d'un individu ou d'un animal par sudation, comportant :
- une face d'application destinée à être appliquée sur la peau de l'individu ou de l'animal ;
- une structure absorbante pour absorber la sueur ;
- une structure intermédiaire située entre la face d'application et la structure absorbante et agencée pour privilégier la diffusion de la sueur depuis la face d'application vers une ou plusieurs zones d'admission, d'étendue restreinte, de la structure absorbante ; et
- un système de détection sensible à l'étendue de la structure absorbante mouillée par la sueur ayant pénétré dans celle-ci par la ou les zones d'admission précitées.

De préférence, le dispositif d'évaluation de la perte hydrique d'un individu ou d'un animal par sudation comporte plus particulièrement :
- une face d'application destinée à être appliquée sur la peau de l'individu ou de l'animal ;
- une structure absorbante pour absorber la sueur, formée en tout ou partie d'un matériau absorbant présentant une capacité d'absorption supérieure ou égale à 500 g/m² ;
- une structure intermédiaire située entre la face d'application et la structure absorbante et agencée pour privilégier la diffusion de la sueur depuis la face d'application vers une ou plusieurs zones d'admission, d'étendue restreinte, de la structure absorbante,
   ladite structure intermédiaire comportant :
   - un film formant barrière à la sueur et traversé par une ou plusieurs ouvertures en correspondance avec la ou lesdites zones d'admission ; et
   - entre le film formant barrière et la face d'application, une structure drainant la sueur formée, en tout ou partie, d'un matériau drainant présentant une capacité d'absorption strictement inférieure à 500 g/m² ; et
- un système de détection sensible à l'étendue de la structure absorbante mouillée par la sueur ayant pénétré dans celle-ci par la ou les zones d'admission précitées.

Le dispositif selon l'invention est destiné à une utilisation embarquée sur tout individu ou animal possédant des glandes sudoripares et apte à transpirer. On peut désigner le dispositif selon l'invention, indifféremment de « dispositif ou système porté » ou encore « patch ».

Le dispositif porté selon l'invention est plus particulièrement distinct d'un pansement. En particulier, il n'est pas destiné à être appliqué sur une plaie.

Par zone « d'étendue restreinte », il faut comprendre que l'étendue de la zone est inférieure à celle de la structure absorbante.

A la différence des dispositifs décrits dans les documents JP 2010-046196 et FR 2 968 532 précités, un dispositif selon l'invention comporte une structure intermédiaire pour la collecte de la sueur, séparée de la zone d'absorption et de mesure. Il est ainsi possible, pour un tel dispositif selon l'invention, d'optimiser à la fois les matériaux de collecte de la sueur, en particulier en utilisant un matériau drainant, comme détaillé dans la suite du texte, et les matériaux absorbants utilisés pour la zone de mesure. Un tel agencement du dispositif selon l'invention permet avantageusement une collecte rapide de la sueur, vers une zone de mesure qui assure un étalement reproductible de la sueur collectée indépendamment de la durée d'utilisation.

Le dispositif selon l'invention permet avantageusement de détecter le niveau de remplissage de la structure absorbante de manière surfacique et de connaître l'évolution de la surface humide, et donc de la quantité de sueur émise dans le temps. La surface mesurée comme humide correspond à la surface réelle de la structure absorbante qui est humide.

Le dispositif selon l'invention permet ainsi d'évaluer la perte hydrique de l'individu ou de l'animal par sudation, c'est-à-dire de déterminer la quantité de sueur transpirée par l'individu ou l'animal sur un temps donné.

Ainsi, la présente invention vise, selon un autre de ses aspects, l'utilisation d'un dispositif tel que décrit précédemment, pour évaluer la perte hydrique par sudation d'un individu ou d'un animal.

L'utilisation du dispositif est réalisée, de préférence, en continu et en temps réel, pour une lecture directe de la quantité de la sueur excrétée par l'individu ou l'animal.

La présente invention se rapporte encore, selon un autre de ses aspects, à un procédé d'évaluation de la perte hydrique d'un individu ou d'un animal par sudation à l'aide d'un dispositif porté tel que défini précédemment, dans lequel :
(i) on applique un tel dispositif sur une zone localisée de la peau de l'individu ou de l'animal ;
(ii) on détecte après un temps donné, grâce au système de détection, l'étendue de la structure absorbante mouillée par la sueur ; et
(iii) on détermine en fonction de cette étendue la quantité de sueur reçue par le dispositif porté.

D'autres caractéristiques, avantages et modes d'application du dispositif selon l'invention, et de son utilisation, ressortiront mieux à la lecture de la description détaillée qui va suivre.

Dans la suite du texte, les expressions « compris entre ... et ... », « allant de ... à ... » et « variant de ... à ... » sont équivalentes et entendent signifier que les bornes sont incluses, sauf mention contraire.

Sauf indication contraire, l'expression « comportant/comprenant un(e) » doit être comprise comme « comportant/comprenant au moins un(e) ».

Selon un mode de réalisation particulièrement préféré, comme évoqué précédemment, la structure intermédiaire du dispositif porté selon l'invention comporte un film formant barrière à la sueur, traversé par une ou plusieurs ouvertures en correspondance avec la ou lesdites zones d'admission, notamment entre 1 et 10 ouvertures. En variante, ou additionnellement, la structure intermédiaire privilégie la diffusion de la sueur vers une ou plusieurs zones d'admission grâce à une anisotropie du ou des matériaux qui la constitue ou à un traitement, par exemple d'imprégnation, qui obture les pores sur la face de la structure intermédiaire en regard de la structure absorbante. On peut encore obstruer les pores de la structure absorbante sur sa face tournée vers la peau, à l'exception de la ou des zones d'admission.

La ou l'ensemble desdites zones d'admission correspondent de préférence à moins de 20 % de la surface totale de la structure absorbante, en particulier moins de 5 % de la surface totale de la structure absorbante.

Le film formant barrière, lorsque présent, est de préférence hydrophobe. Le film formant barrière est de préférence en un matériau semi-perméable, étant par exemple en polyuréthane. Par semi-perméable, on entend un matériau perméable à un gaz, tel que l'air et imperméable à un liquide.

L'homme du métier est à même de mettre en oeuvre un système de détection approprié à l'évaluation de l'étendue de la structure absorbante mouillée par la sueur.

Le système de détection comporte de préférence au moins un réseau d'électrodes, notamment deux réseaux d'électrodes agencés sous forme de grille. Le ou les réseaux d'électrodes sont isolés électriquement entre eux, lorsque les électrodes se croisent. Lorsque le système de détection comporte deux réseaux d'électrodes agencés sous forme de grille, des matrices ligne et colonne peuvent être construites avec les valeurs des mesures entre les couples d'électrodes consécutives de chaque réseau, et un produit matriciel des matrices ligne et colonne effectué pour aboutir à une matrice résultat dont les coefficients sont représentatifs du degré d'humidité en divers emplacements de la structure absorbante dont les coordonnées sont associées aux coefficients des matrices ligne et colonne.

Il est alors possible de générer une information signalant non seulement la préférence d'humidité ou non à tel emplacement mais également de quantifier le degré d'humidité de chaque emplacement, ce qui peut servir de base à une représentation cartographique où les degrés d'humidité sont signalés par des couleurs différentes.

Les électrodes peuvent être portées par un support ne gênant pas la diffusion de la sueur et non occlusif. Ce support peut être figuré, par exemple, par un film hydrophobe, tel que par exemple un film non tissé hydrophobe. Cela permet d'éviter que la sueur ne s'étale entre les différentes électrodes.

Le support des électrodes peut, le cas échéant, être le film formant barrière ou la structure absorbante elle-même, ou une enveloppe externe du dispositif porté.

L'agencement des électrodes peut être tel qu'il ne nécessite pas de disposer les électrodes sur un support, ce qui est le cas par exemple lorsque les électrodes sont disposées selon une grille.

Le système de détection comporte de préférence plusieurs emplacements de mesure répartis sur la structure absorbante, de façon à obtenir de préférence entre 2 et 100 emplacements de mesure, en particulier de 10 à 50 emplacements de mesure et plus particulièrement de 30 à 50 emplacements de mesure. Un emplacement de mesure peut être localisé entre deux électrodes consécutives du réseau d'électrodes. La mesure peut être effectuée sur sensiblement toute la longueur de ces électrodes au contact de la structure absorbante.

Les emplacements de mesure peuvent être répartis de façon régulière ou non sur la structure absorbante. Il peut s'avérer préférable de disposer d'emplacements de mesure plus resserrés lorsque l'on est loin de la ou des zones d'admission afin de bénéficier d'une résolution spatiale meilleure loin de la ou des zones d'admission, et de gagner ainsi en précision. Par exemple, l'écart entre les électrodes du réseau est plus faible à distance de la ou des zones d'admission qu'à proximité. Les électrodes peuvent être rectilignes ou non. Par exemple, dans le cas d'un agencement sous forme de grille, au moins certaines électrodes peuvent être curvilignes et contourner la ou les zones d'admission.

Les électrodes peuvent être filaires ou réalisées par sérigraphie, gravure ou métallisation. Lorsque les électrodes forment une grille, elles peuvent être reliées les unes aux autres à leurs intersections par un isolant électrique qui assure leur fixation.

Le système de détection peut être relié à un système de mesure qui détermine par exemple l'impédance entre deux électrodes consécutives, pour au moins plusieurs des couples d'électrodes consécutives pouvant être générés. Par exemple, le système de mesure injecte une tension alternative entre deux électrodes consécutives et mesure l'amplitude du courant circulant dans ces électrodes, ce courant étant par exemple déterminé par mesure de la tension de crête aux bornes d'une résistance parcourue par ce courant. Le système de détection peut être relié au système de mesure par une ou plusieurs connexions, de telle sorte que le système de mesure est aisément déconnectable du système de détection.

La tension alternative est par exemple un signal basse fréquence, de fréquence inférieure à 25 kHz. Le signal est par exemple de forme d'onde en créneaux. L'amplitude de la tension injectée est par exemple inférieure ou égale à 5 V.

Le système de détection couvre de préférence une surface de détection qui correspond à au moins 50 % de la surface totale de la structure absorbante.

Le système de détection peut être au contact d'une face de la structure absorbante opposée à la structure intermédiaire. En variante, le système de détection est au contact d'une face de la structure absorbante située du côté de la structure intermédiaire. Ces deux variantes peuvent être combinées, le système de détection étant alors situé de part et d'autre de la structure absorbante.

La ou les zones d'admission précitées occupent de préférence une position centrale relativement à la structure absorbante. En variante, la ou les zones d'admission occupent une position excentrée relativement à la structure absorbante. Par exemple, le dispositif porté comporte une zone unique d'arrivée de la sueur dans la structure absorbante, située à proximité de sa périphérie, par exemple dans un coin pour un dispositif de contour polygonal, notamment carré, ou en variante autant de zones d'admission qu'il y a de coins, disposées chacune dans un coin.

Selon un mode de réalisation particulièrement préféré, la structure absorbante du dispositif selon l'invention est formée, en tout ou partie, d'un matériau dit absorbant, c'est-à-dire présentant une capacité d'absorption supérieure ou égale à 500 g/m², de préférence supérieure ou égale à 800 g/m².

La capacité d'absorption peut être par exemple obtenue par pesée différentielle entre le poids du matériau gorgé d'eau à saturation, et celui du même matériau sec.

Un matériau absorbant possède une capacité d'absorption plus élevée qu'un matériau dit drainant, tel que mis en oeuvre dans la structure drainante du dispositif décrite ci-dessous.

Avantageusement, l'étalement d'un liquide, comme la sueur, absorbé dans un matériau absorbant est réduit dans le temps, voire indépendant du temps. Ainsi, la quantité de sueur absorbée dans la structure absorbante occupe avantageusement un volume de la structure absorbante donné, ce même volume ne s'étalant pas dans un volume supérieur de la structure absorbante au cours du temps.

A titre d'exemple, la structure absorbante peut être formée en tout ou partie d'un matériau alvéolaire, notamment à base de mousse de polyuréthane à cellules ouvertes, de mousse de polymère(s) superabsorbant(s) (SAP), comme par exemple de polyacrylates et/ou d'un matériau fibreux, par exemple à base de fibres de cellulose.

La structure absorbante peut également comporter un ou plusieurs agents superabsorbants, par exemple des polyacrylates.

Comme évoqué précédemment, la structure intermédiaire du dispositif porté selon l'invention comporte avantageusement, entre le film formant barrière et la face d'application, une structure drainant la sueur transpirée par la peau.

Selon un mode de réalisation particulièrement préféré, la structure drainant la sueur est formée, en tout ou partie, d'un matériau dit drainant, c'est-à-dire présentant une capacité d'absorption strictement inférieure à 500 g/m², en particulier comprise entre 30 et 500 g/m².

La structure drainante peut comporter, voire être formée de, deux couches superposées, à savoir une couche proximale du côté de la peau, drainant axialement la sueur, et une couche distale qui lui est superposée, drainant transversalement la sueur.

La couche proximale drainant axialement la sueur peut être formée en tout ou partie d'un matériau alvéolaire, tel qu'une mousse de polyuréthane à cellules ouvertes par exemple.

La couche distale drainant transversalement la sueur peut être formée en tout ou partie d'un matériau non tissé ou d'un tricot, notamment de viscose.

Comme précisé précédemment, l'invention a encore pour objet un procédé d'évaluation de la perte hydrique d'un individu ou d'un animal par sudation, comprenant au moins les étapes suivantes:
(i) on applique un dispositif selon l'invention sur une zone localisée de la peau de l'individu ou de l'animal ;
(ii) on détecte après un temps donné, grâce au système de détection, l'étendue de la structure absorbante mouillée par la sueur ; et
(iii) on détermine en fonction de cette étendue la quantité de sueur reçue par le dispositif porté.

L'étape (ii) peut être répétée pour mesurer en continu la quantité de sueur sécrétée par l'individu ou l'animal.

Le procédé peut comporter l'affichage d'une grandeur représentative de la quantité de sueur absorbée par la structure absorbante, du débit de sueur sécrétée par l'individu et/ou le déclenchement d'une alarme lorsque la quantité de sueur absorbée atteint une limite prédéfinie.

L'étape (ii) de détection de l'étendue de la structure absorbante mouillée par la sueur peut comprendre la mesure d'une grandeur électrique représentative de l'impédance électrique entre deux électrodes du système de détection, à l'aide d'un dispositif de mesure relié à celui-ci, notamment à deux électrodes consécutives.

L'étape (ii) peut être répétée pour mesurer successivement ladite grandeur électrique entre différentes paires d'électrodes successives du ou de chaque réseau d'électrodes.

Le système de mesure peut être externe au dispositif porté selon l'invention et les connexions avec le système de détection peuvent être établies avant le positionnement du dispositif sur la peau de l'individu ou de l'animal. En variante, le système de mesure est intégré au dispositif, grâce par exemple à un circuit électrique auquel sont reliées les électrodes, qui effectue les mesures aux bornes des électrodes. Le circuit électronique peut transmettre les résultats de la mesure à un dispositif d'affichage externe au dispositif porté. En variante, le circuit électronique comporte une puce RFID qui est interrogée. Dans ce cas, l'énergie nécessaire au fonctionnement du système de mesure peut être fournie par couplage inductif par le lecteur utilisé.

Dans le cas où le système de mesure est externe, le dispositif porté selon l'invention peut comporter un identifiant qui est détecté par le système de mesure. Cela permet à ce dernier de sélectionner par exemple des données en mémoire adaptées au dispositif identifié, concernant par exemple le système de détection présent et la nature de la structure absorbante, afin de pouvoir en tenir compte pour la mesure.

Le système de mesure peut également être agencé pour vérifier, avant la poursuite des mesures, que la structure absorbante est bien sèche et que le système de détection n'a pas été endommagé. Le cas échéant, des mesures sont effectuées afin de servir de référence pour la suite.

La mesure de l'évolution de l'étendue de la structure absorbante mouillée par la sueur permet de déterminer, en fonction du temps, le volume de sueur émise au niveau de la zone d'application du dispositif sur la peau et, par conséquent, d'en déduire la perte hydrique de l'individu ou de l'animal par sudation.

L'étendue de la structure absorbante mouillée est représentative du volume de sueur absorbé par la structure absorbante.

La corrélation entre la quantité de sueur reçue par le dispositif et l'étendue de la structure absorbante mouillée peut être réalisée classiquement par étalonnage, préalablement à l'évaluation de la perte hydrique d'un individu ou d'un animal à l'aide du dispositif.

Par étalonnage, on entend la réalisation au préalable d'une ou plusieurs courbes d'étalonnage dans des conditions similaires, et pour des quantités connues de sueur ou d'un liquide comme l'eau ayant des propriétés physico-chimiques proches ou similaires à celles de la sueur. Les valeurs d'étendue de la structure absorbante obtenues à partir du dispositif embarqué sur l'individu ou l'animal peuvent être comparées aux courbes d'étalonnage pour déterminer la quantité de sueur absorbée.

Les résultats obtenus peuvent également être extrapolés pour être ramenés à une perte hydrique globale. L'extrapolation peut tenir compte, notamment, du rapport surfacique entre la zone localisée de la peau recouverte par le dispositif porté et la surface totale de la peau du corps. Elle peut également tenir compte de la position du dispositif porté sur le corps humain, certaines zones du corps, notamment la cuisse, étant préférées pour le positionnement du dispositif.

L'invention pourra être mieux comprise à la lecture de la description détaillée qui va suivre, d'exemples non limitatifs de mise en oeuvre de celle-ci, et à l'examen des dessins annexés, sur lesquels :
- la figure 1 est une vue schématique, en coupe, d'un exemple de dispositif d'évaluation de la perte hydrique d'un individu ou d'un animal par sudation, conforme à l'invention, relié à un système de mesure externe,
- la figure 2 est une vue analogue à la figure 1 d'une variante de réalisation du dispositif porté,
- la figure 3 représente un exemple d'agencement des électrodes au sein d'un système de détection,
- la figure 4 illustre l'utilisation du système de détection de la figure 3 pour déterminer l'étendue de la surface mouillée de la structure absorbante,
- les figures 5 à 7 sont des vues analogues à la figure 3, de variantes de réalisation du système de détection.

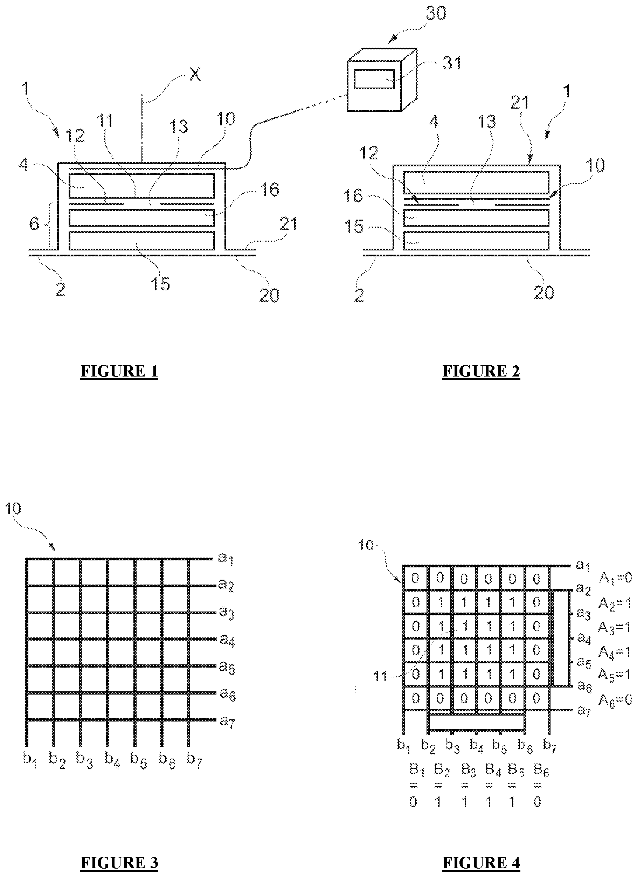

Sur les figures 1 et 2, les proportions réelles des différents éléments constitutifs n'ont pas toujours été respectées, dans un souci de clarté du dessin.

On a représenté à la figure 1 un exemple de dispositif porté 1 selon l'invention, relié à un système de mesure 30 qui comporte par exemple un écran 31 permettant d'afficher des informations liées à l'utilisation du dispositif, notamment le volume de sueur absorbée.

Dans l'exemple illustré, le dispositif porté 1 est relié par une liaison filaire au système de mesure 30, mais en variante la transmission des informations s'effectue par une liaison sans fil entre le dispositif 1 et le système de mesure 30. Dans ce cas, le dispositif porté est équipé d'un circuit électronique qui effectue les mesures et les transmet au système de mesure, lequel peut assurer une partie des calculs ou n'avoir qu'une fonction d'affichage. Le système de mesure peut aussi éventuellement fournir, par couplage inductif, l'énergie nécessaire au fonctionnement du circuit électronique.

Le dispositif 1 présente une face 2 d'application sur la peau et intègre une structure absorbante 4 prévue pour absorber la sueur sécrétée.

Le dispositif 1 comporte également une structure intermédiaire 6, située entre la face d'application 2 et la structure absorbante 4.

Un système de détection 10 est situé au contact de la structure absorbante 4, par exemple au-dessus de celle-ci, comme illustré à la figure 1.

Le système de détection 10 est sensible à l'étendue de la structure absorbante mouillée par la sueur ayant pénétré dans celle-ci.

De façon à privilégier la diffusion de la sueur depuis la face d'application 2 vers une zone d'admission 11 d'étendue restreinte de la structure absorbante, la structure intermédiaire 6 comporte dans l'exemple illustré un film formant barrière 12 aux liquides, en particulier la sueur, disposé au contact de la face inférieure de la structure absorbante 4.

Ce film formant barrière 12 est ajouré en regard de la zone d'admission 11 et présente au moins une ouverture 13 à cet effet. Par ouverture, on entend une zone permettant le passage de la sueur de part et d'autre du film formant barrière 12. Il peut s'agir d'un trou ou d'une zone poreuse. A titre d'exemple, le film formant barrière 12 peut être un film de polyuréthane perforé en son centre de 4 trous de diamètre 5 mm. On comprend que dans ces alternatives, la sueur est apte à passer de part et d'autre du film formant barrière 12.

La structure intermédiaire 6 peut comporter, entre le film 12 formant barrière et la face d'application 2, une structure drainante composée d'une couche proximale 15 drainant axialement la sueur, c'est-à-dire sensiblement selon l'axe X perpendiculaire à la peau, vertical sur la figure 1, et d'une couche distale 16 drainant transversalement la sueur, c'est-à-dire sensiblement perpendiculairement à l'axe X, soit horizontalement sur la figure 1.

La couche proximale 15 est de préférence, comme illustré, adjacente à la face d'application 2. Cette dernière peut être définie par un voile de contact 20 d'un matériau adapté à être en contact avec la peau. Ce matériau est inerte vis-à-vis de la sueur et, de préférence, dermatologiquement acceptable.

Le dispositif porté peut comporter une enveloppe externe 21 qui est fixée au voile 20 et qui recouvre les couches 15 et 16, le film 12, la structure absorbante 4 et le système de détection 10. L'enveloppe 21 peut être réalisée dans un matériau semi-perméable, laissant passer l'air.

L'enveloppe 21 peut être fixée au voile 20 à la périphérie du dispositif porté 1, par exemple par soudure ou collage.

Le dispositif peut comporter avantageusement des moyens de fixation du dispositif sur la peau, non représentés en figures 1 et 2. Par exemple, le dispositif porté peut être maintenu sur la zone localisée de la peau où la mesure doit être effectuée à l'aide d'un ou plusieurs adhésifs situés en périphérie du dispositif. Les moyens de fixation peuvent encore être constitués par des attaches destinées à ceinturer une partie du corps de l'individu ou de l'animal. Les attaches peuvent être tout organe de serrage mécanique connu apte à maintenir le dispositif autour d'une partie du corps de l'individu ou de l'animal. On peut citer, à titre d'exemple, les bandes de type Velcro^{®}, les lanières, les bracelets ou les ceintures dorsales.

La couche drainante proximale 15 peut être réalisée dans un matériau alvéolaire, par exemple une mousse de polyuréthane à cellules ouvertes.

La couche drainante distale 16 est par exemple réalisée dans un matériau dit non tissé, notamment de cellulose, et n'être par exemple qu'en cellulose.

Lorsque le dispositif porté 1 est en place sur la peau, la sueur qui remonte dans la structure intermédiaire 6 pénètre dans la structure absorbante 4 de façon localisée, au travers de l'ajour 13. Ainsi, l'admission de la sueur se fait par la ou les zones 11 en regard de l'ajour 13.

Dans l'exemple de la figure 1, le système de détection 10 n'est pas au contact du film 12, étant situé entre la structure absorbante 4 et le dessus de l'enveloppe 21. En variante, le système de détection 10 est situé au contact du film 12, entre ce dernier et la structure absorbante 4, comme illustré à la figure 2. Dans ce cas, le film 12 peut servir de support aux électrodes du système de détection, et par exemple recevoir des pistes conductrices déposées par impression ou métallisation sélective. En cas notamment d'impression des électrodes et de croisement de celles-ci, un isolant peut être déposé localement aux intersections, entre les électrodes.

Dans le cas où le système de détection est couplé à un système de mesure intégré au dispositif porté, une antenne peut également être réalisée sur le support des électrodes. Le système de détection peut être couplé au système de mesure au moyen de connecteurs disposés sur les électrodes, ces derniers permettant une connexion réversible avec le système de mesure.

Le choix du positionnement du système de détection peut se faire, le cas échéant, en fonction de la nature du matériau constituant la structure absorbante, de façon à avoir les meilleurs résultats et/ou la plus grande facilité de fabrication. Ainsi, les deux réseaux d'électrodes peuvent être avantageusement respectivement situés de part et d'autre de la structure absorbante ; ce mode de réalisation permet d'estimer la distribution de la sueur à différents endroits dans la structure absorbante.

L'intérêt d'avoir un ajour 13 central constitué d'un ou de plusieurs trous rapprochés est d'avoir une progression concentrique de la sueur au sein de la structure absorbante 4 qui s'humidifie, ce qui réduit le risque d'avoir une information faussée sur l'étendue de la structure absorbante mouillée par la sueur, et donc sur la quantité de sueur absorbée par la structure absorbante.

Le système de détection 10 est réalisé de façon à permettre des mesures en plusieurs emplacements, afin de déterminer l'étendue de la structure absorbante mouillée par la sueur.

Pour réaliser la mesure en plusieurs emplacements, le système de détection peut comporter au moins un réseau d'électrodes, chaque électrode occupant une position connue relativement à la ou aux zones d'admission 11 à partir de laquelle ou desquelles la structure absorbante 4 se remplit de sueur, compte tenu de la présence de l'ajour 13.

Ainsi, la distance des électrodes du réseau à chaque zone d'admission 11 varie, de façon régulière ou non. L'écartement des électrodes au sein d'un réseau peut varier et par exemple diminuer lorsque l'on s'éloigne d'une zone d'admission 11, pour bénéficier d'une meilleure précision lorsque l'étendue de la structure absorbante mouillée par la sueur est proche du maximum.

De préférence, le système de détection 10 comporte au moins deux réseaux d'électrodes qui sont réparties dans deux directions différentes du plan selon lequel s'étend la structure absorbante 4, par exemple deux directions perpendiculaires entre elles.

La présence de deux réseaux d'électrodes aᵢ et bⱼ disposés selon une grille est particulièrement avantageuse en ce qu'elle permet d'obtenir un nombre élevé d'emplacements de mesure, mais l'invention n'est pas limitée à une disposition particulière des électrodes, pourvu que l'on puisse obtenir une information sur l'étendue de la structure absorbante 4 mouillée en interrogeant le système de détection 10. Par emplacement de mesure, on entend une zone de la structure absorbante 4 délimitée par des électrodes aᵢ, bⱼ. Dans l'exemple considéré, chaque emplacement de mesure correspond à une maille de la grille.

La figure 3 représente un exemple de système de détection 10 comportant des réseaux d'électrodes aᵢ et bᵢ agencés selon une grille. Sur cette figure, le système de détection comporte un premier réseau d'électrodes a₁, ... aₙ et un deuxième réseau d'électrodes b₁ ... bₙ, avec n égal à sept par exemple, comme illustré.

Les électrodes sont des conducteurs électriques qui se croisent sans se toucher mais sont électriquement en contact en plusieurs points de leur longueur avec la structure absorbante 4. Quand la structure absorbante 4 est localement chargée de sueur entre deux électrodes adjacentes aⱼ, aⱼ₊₁, l'impédance entre ces électrodes change et ce changement peut être détecté par une mesure d'une grandeur électrique aux bornes des électrodes. Il en est de même lorsque deux électrodes adjacentes bⱼ, bⱼ₊₁ recouvrent une zone localement chargée de sueur.

Les électrodes peuvent être isolées aux croisements par un adhésif de type scotch Kapton^{™} ce qui permet d'obtenir un ensemble d'électrodes autoporteur, c'est-à-dire que le maintien de la position relative des électrodes d'un réseau est assuré par les électrodes de l'autre réseau, et inversement. De plus, le système de détection est de cette façon assez souple, ce qui lui permet de s'adapter facilement à la surface sur laquelle il est appliqué.

L'écart entre les électrodes de chacun des réseaux est par exemple de 15 mm, la largeur d'une électrode est par exemple de 2 mm et la taille de la grille est par exemple de 70 mm par 70 mm.

A titre d'exemple, sur la figure 4, l'état des mesures effectuées entre différents couples d'électrodes a été représenté, l'ajour 13 étant constitué ici par quatre trous centraux, définissant autant de zones d'admission 11.

L'évaluation du remplissage de la structure absorbante 4 peut consister, comme dans cet exemple, à mesurer la variation de la conductivité électrique entre deux électrodes successives aᵢ ou bⱼ. Dans un exemple, lorsque deux électrodes successives sont en contact électrique en raison de la présence de sueur absorbée par la structure absorbante 4 entre elles, les paramètres Aᵢ ou Bⱼ valent 1. L'étendue de la structure absorbante mouillée par la sueur peut alors être estimée à partir du produit de la matrice colonne A(A1,A2,A3,A4,A5,A6) par la matrice ligne B(B1,B2,B3,B4,B5,B6).

Un exemple d'estimation de l'étendue de la structure absorbante mouillée par la sueur est présenté à la figure 4, où en raison de la présence de sueur entre les électrodes a₂, a₃, a₄, a₅ et a₆, on obtient la matrice colonne A(A1,A2,A3,A4,A5,A6) qui vaut (0,1,1,1,1,0) et en raison de la présence de sueur entre les électrodes b₂, b₃, b₄, b₅ et b₆, on obtient la matrice ligne B(B1,B2,B3,B4,B5,B6) qui vaut (0,1,1,1,1,0).

L'étendue de la structure absorbante mouillée par la sueur est évaluée dans cet exemple à partir d'une matrice d'humidité C(i,j), déterminée par le produit de la matrice colonne A par la matrice ligne B. Les coefficients de la matrice C(i,j) qui valent 1 représentent chacun une zone de la structure absorbante qui est humide, et ceux qui valent 0 une zone de la structure absorbante qui est encore sèche.

A partir de ces zones mesurées comme humides, le volume de sueur absorbée par la structure absorbante peut être visualisé de plusieurs manières, à l'aide du système de mesure 30 auquel est relié le système de détection 10.

Le résultat peut être exprimé sous la forme d'une valeur affichée indiquant par exemple le débit de sueur transpirée par la peau.

L'invention n'est pas limitée à un système de détection 10 présentant une structure particulière.

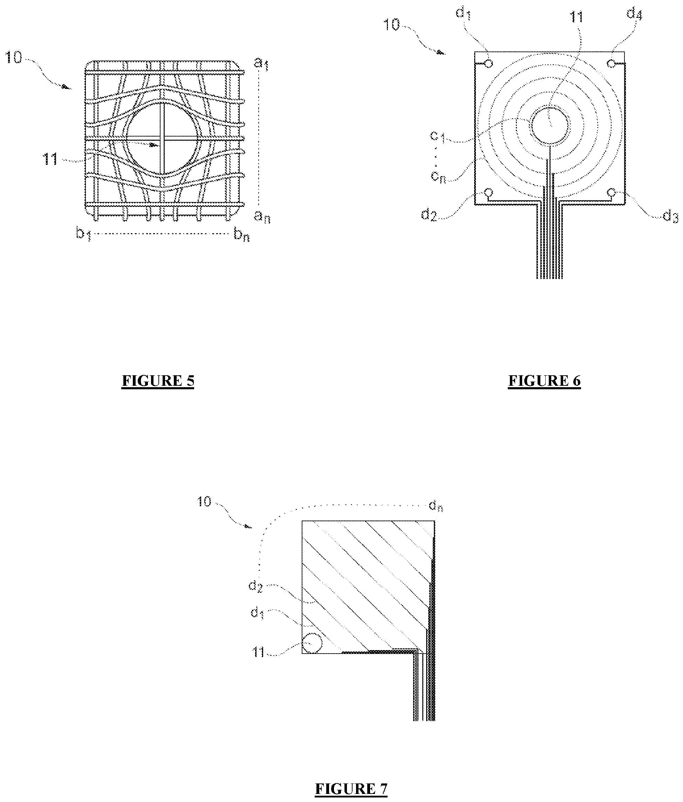

Une variante du système de détection 10 est ainsi illustrée à la figure 5. L'objectif de la géométrie présentée à la figure 5 est d'avoir une meilleure estimation de l'étendue de la structure absorbante mouillée par la sueur, lorsque celle-ci est proche de sa valeur maximale. Le principe consiste à écarter les électrodes de la zone d'admission, au centre de la grille, comme si les électrodes devaient contourner un cylindre virtuel placé au centre la grille. Seules les deux électrodes centrales restent rectilignes et l'écart entre deux électrodes successives d'un même réseau d'électrodes est réduit au niveau de l'intersection avec l'électrode de l'autre réseau restée rectiligne.

La variante illustrée à la figure 6 comporte un réseau d'électrodes concentriques c₁, ..., cₙ disposées autour de la zone d'admission 11, centrale, ainsi que des électrodes périphériques d₁, ..., d₄ qui aboutissent dans le voisinage de chacun des coins de la structure absorbante 4. La mesure peut s'effectuer entre chaque couple cⱼ, cⱼ₊₁ d'électrodes consécutives, et entre l'électrode cₙ radialement la plus extérieure et chacune des électrodes de coin d₁ à d₄. Les conducteurs d'alimentation des électrodes c₁ à cₙ et d₁ à d₄ peuvent être électriquement isolés de la structure absorbante afin que la mesure s'effectue bien au niveau des électrodes.

La variante illustrée à la figure 7 se caractérise par le fait que le remplissage s'effectue à partir d'un coin, la zone d'admission 11 étant excentrée.

Les électrodes d₁, ..., dₙ sont par exemple disposées, comme illustré, à partir de ce coin en direction du coin opposé, étant par exemple parallèles entre elles et parallèles à la diagonale reliant les deux autres coins. Ainsi, les électrodes sont orientées sensiblement transversalement à la direction dans laquelle se propage la sueur dans la structure absorbante lors du remplissage de celle-ci. La mesure peut s'effectuer entre chaque couple dⱼ,dⱼ₊₁ d'électrodes adjacentes.

L'invention n'est pas limitée aux exemples illustrés. Par exemple, encore d'autres agencements d'électrodes peuvent être utilisés.

## Revendications

1. Dispositif (1) d'évaluation de la perte hydrique d'un individu ou d'un animal par sudation, comportant :
- une face d'application (2) destinée à être appliquée sur la peau de l'individu ou de l'animal ;
- une structure absorbante (4) pour absorber la sueur, ladite structure absorbante étant formée en tout ou partie d'un matériau absorbant présentant une capacité d'absorption supérieure ou égale à 500 g/m² ;
- une structure intermédiaire (6) située entre la face d'application (2) et la structure absorbante (4) et agencée pour privilégier la diffusion de la sueur depuis la face d'application (2) vers une ou plusieurs zones d'admission (11), d'étendue restreinte, de la structure absorbante (4) ; et
- un système de détection (10) sensible à l'étendue de la structure absorbante mouillée par la sueur ayant pénétré dans celle-ci par la ou les zones d'admission (11),
ledit dispositif comportant entre la face (2) d'application sur la peau et la structure absorbante (4), une structure drainant la sueur transpirée par la peau formée en tout ou partie d'un matériau drainant présentant une capacité d'absorption strictement inférieure à 500 g/m².

2. Dispositif selon la revendication 1, la structure intermédiaire (6) comportant un film (12) formant barrière à la sueur, traversé par une ou plusieurs ouvertures (13) en correspondance avec la ou les zones d'admission (11), notamment entre 1 et 10 ouvertures.

3. Dispositif selon la revendication 1 ou 2, la ou les zones d'admission (11) correspondant à moins de 20 % de la surface totale de la structure absorbante (4), en particulier moins de 5 % de la surface totale de la structure absorbante.

4. Dispositif selon la revendication 2 ou 3, le film (12) formant barrière étant hydrophobe.

5. Dispositif selon l'une quelconque des revendications 2 à 4, le film (12) formant barrière étant en un matériau semi-perméable, par exemple en polyuréthane.

6. Dispositif selon l'une quelconque des revendications précédentes, le système de détection (10) comportant un ou plusieurs réseaux d'électrodes, en particulier deux réseaux d'électrodes (a₁,...aₙ; b₁,..., bₙ) agencés sous forme de grille.

7. Dispositif selon l'une quelconque des revendications précédentes, le système de détection (10) comportant plusieurs emplacements de mesure répartis sur la structure absorbante, de préférence entre 2 et 100 emplacements de mesure, en particulier de 10 à 50 emplacements de mesure.

8. Dispositif selon l'une quelconque des revendications précédentes, le système de détection (10) couvrant une surface de détection qui correspond à au moins 50 % de la surface totale de la structure absorbante.

9. Dispositif selon l'une quelconque des revendications précédentes, la structure absorbante (4) étant formée en tout ou partie d'un matériau absorbant, présentant une capacité supérieure ou égale à 800 g/m².

10. Dispositif selon l'une quelconque des revendications précédentes, la structure absorbante (4) étant formée en tout ou partie d'un matériau alvéolaire, notamment à base de mousse de polyuréthane à cellules ouvertes, de mousse de polymère(s) superabsorbant(s) notamment de polyacrylates ou d'un matériau fibreux, notamment à base de fibres de cellulose.

11. Dispositif selon l'une quelconque des revendications précédentes, la structure drainant la sueur étant formée en tout ou partie d'un matériau drainant, présentant une capacité d'absorption comprise entre 30 et 500 g/m².

12. Dispositif selon l'une quelconque des revendications précédentes, la structure drainante comportant deux couches superposées, la couche proximale (15) du côté de la peau drainant axialement la sueur et la couche distale (16) qui lui est superposée drainant transversalement la sueur.

13. Dispositif selon la revendication précédente, la couche proximale (15) étant formée en tout ou partie d'un matériau alvéolaire, notamment de mousse de polyuréthane à cellules ouvertes et/ou la couche drainante distale (16) étant formée en tout ou partie d'un matériau non tissé ou d'un tricot, notamment de viscose.

14. Utilisation d'un dispositif (1) tel que défini selon l'une quelconque des revendications 1 à 13, pour évaluer la perte hydrique par sudation d'un individu ou d'un animal.

15. Procédé d'évaluation de la perte hydrique d'un individu ou d'un animal par sudation, dans lequel :
(i) on applique un dispositif selon l'une quelconque des revendications 1 à 13 sur une zone localisée de la peau de l'individu ou de l'animal ;
(ii) on détecte après un temps donné, grâce au système de détection, l'étendue de la structure absorbante mouillée par la sueur ; et
(iii) on détermine en fonction de cette étendue la quantité de sueur reçue par le dispositif porté.

16. Procédé selon la revendication précédente, dans lequel le système de détection (10) est tel que défini selon la revendication 6, et l'étape (ii) comprend la mesure d'une grandeur électrique représentative de l'impédance électrique entre deux électrodes du système de détection à l'aide d'un système de mesure (30), en particulier l'étape (ii) étant répétée pour mesurer ladite grandeur électrique entre différentes paires d'électrodes successives du ou de chaque réseau d'électrodes.

## Patentansprüche

1. Vorrichtung (1) zur Bewertung des Wasserverlustes einer Person oder eines Tieres durch Schwitzen, mit:
- einer Applikationsfläche (2), die dazu bestimmt ist, auf die Haut der Person oder des Tieres aufgelegt zu werden,
- einer absorbierenden Struktur (4) zum Absorbieren des Schweißes, welche absorbierende Struktur ganz oder zum Teil aus einem absorbierenden Material hergestellt ist, das eine Absorptionskapazität größer oder gleich 500 g/m² hat,
- einer Zwischenstruktur (6), die zwischen der Applikationsfläche (2) und der absorbierenden Struktur (4) angeordnet und dazu ausgebildet ist, die Diffusion des Schweißes von der Applikationsfläche (2) zu einer oder mehreren Einlasszonen (11), von begrenzter Ausdehnung, der absorbierenden Struktur (4) zu begünstigen, und
- einem Detektionssystem (10), das für die Ausdehnung der absorbierenden Struktur empfindlich ist, die durch den Schweiß angefeuchtet wurde, der über die Einlasszone oder Einlasszonen (11) in sie eingedrungen ist, wobei die genannte Vorrichtung zwischen der Applikationsfläche (2) und der absorbierenden Struktur (4) eine Struktur zur Drainage des von der Haut abgegebenen Schweißes aufweist, die ganz oder zum Teil aus einem Drainagematerial hergestellt ist, das eine Absorptionskapazität streng unterhalb von 500 g/m² hat.

2. Vorrichtung nach Anspruch 1, bei der die Zwischenstruktur (6) einen Film (12) aufweist, der eine Schweißbarriere bildet, der von einer oder mehreren Öffnungen (13) durchsetzt ist, die mit der Einlasszone oder den Einlasszonen (11) korrespondieren, insbesondere zwischen 1 und 10 Öffnungen.

3. Vorrichtung nach Anspruch 1 oder 2, bei der die Einlasszone oder Einlasszonen (11) weniger als 20 % der gesamten Oberfläche der absorbierenden Struktur entsprechen, insbesondere weniger als 5 % der gesamten Oberfläche der absorbierenden Struktur.

4. Vorrichtung nach Anspruch 2 oder 3, bei der der Film (12), der die Barriere bildet, hydrophob ist.

5. Vorrichtung nach einem der Ansprüche 2 bis 4, bei der der Film (12), der die Barriere bildet, aus einem semipermeablen Material gebildet ist, beispielsweise aus Polyurethan.

6. Vorrichtung nach einem der vorstehenden Ansprüche, bei der das Detektionssystem (10) ein oder mehrere Elektrodennetze aufweist, insbesondere zwei Elektrodennetze (a₁, ...., aₙ; d₁, ......,dₙ), die in der Form von Gittern ausgebildet sind.

7. Vorrichtung nach einem der vorstehenden Ansprüche, bei der das Detektionssystem mehrere Messstellen aufweist, die auf der absorbierenden Struktur verteilt sind, vorzugsweise zwischen 2 und 100 Messstellen, insbesondere 10 bis 50 Messstellen.

8. Vorrichtung nach einem der vorstehenden Ansprüche, bei der das Detektionssystem (10) eine Detektionsfläche überdeckt, die wenigstens 50 % der gesamten Oberfläche der absorbierenden Struktur entspricht.

9. Vorrichtung nach einem der vorstehenden Ansprüche, bei der die absorbierende Struktur (4) ganz oder zum Teil aus einem absorbierenden Material hergestellt ist, das eine Kapazität größer oder gleich 800 g/m² hat.

10. Vorrichtung nach einem der vorstehenden Ansprüche, bei der die absorbierende Struktur (4) ganz oder zum Teil aus einem zellenförmigen Material hergestellt ist, insbesondere auf der Basis von offenzelligem Polyurethanschaum, superabsorbierendem Schaumpolymer oder superabsorbierenden Schaumpolymeren, insbesondere Polyacrylaten oder einem faserigen Material, insbesondere auf der Basis von Zellulosefasern.

11. Vorrichtung nach einem der vorstehenden Ansprüche, bei der die Drainagestruktur für den Schweiß ganz oder zum Teil aus einem Drainagematerial gebildet ist, das eine Absorptionskapazität zwischen 30 und 500 g/m² hat.

12. Vorrichtung nach einem der vorstehenden Ansprüche, bei der die Drainagestruktur zwei einander überlagerte Schichten aufweist, wobei die proximale Schicht (15) auf der Seite der Haut den Schweiß axial ableitet und die distale Schicht (16), die ihr überlagert ist, den Schweiß transversal ableitet.

13. Vorrichtung nach dem vorstehenden Anspruch, bei der die proximale Schicht (15) ganz oder zum Teil aus einem zellenförmigen Material gebildet ist, insbesondere aus offenzelligem Polyurethanschaum, und/oder die distale Drainageschicht (16) ganz oder zum Teil aus einem nichtgewebten Material oder einem Gestrick gebildet ist, insbesondere aus Viskose.

14. Verwendung einer Vorrichtung (1) nach einem der Ansprüche 1 bis 13 zur Bewertung des Wasserverlustes einer Person oder eines Tieres durch Schwitzen.

15. Verfahren zur Bewertung des Wasserverlustes einer Person oder eines Tieres durch Schwitzen, bei dem:
(i) man eine Vorrichtung nach einem der Ansprüche 1 bis 13 auf eine lokalisiere Zone der Haut der Person oder des Tieres auflegt,
(ii) man nach einer gegebenen Zeit mit Hilfe des Detektionssystems die Ausdehnung der absorbierenden Struktur misst, die durch den Schweiß angefeuchtet wurde, und
(iii) man die Menge des von der getragenen Vorrichtung aufgenommenen Schweißes als Funktion dieser Ausdehnung bestimmt.

16. Verfahren nach dem vorstehenden Anspruch, bei dem das Detektionssystem (10) so ausgebildet ist, wie in Anspruch 6 definiert wird, und der Schritt (ii) die Messung einer elektrischen Größe einschließt, die für die elektrische Impedanz zwischen zwei Elektroden des Detektionssystems repräsentativ ist, mit Hilfe eines Messsystems (30), insbesondere mit Wiederholung des Schrittes (ii), um die genannte elektrische Größe zwischen verschiedenen Paaren aufeinanderfolgender Elektroden des oder jedes Elektrodennetzes zu messen.

## Claims

1. Device (1) for assessing the water loss of a person or of an animal through perspiration, comprising:
- an application face (2) intended to be applied to the skin of the person or of the animal;
- an absorbent structure (4) for absorbing the perspiration, this absorbent structure being formed completely or partly of an absorbent material having an absorption capacity that is greater than or equal to 500 g/m2;
- an intermediate structure (6) situated between the application face (2) and the absorbent structure (4) and arranged to favour distribution of the perspiration from the application face (2) to one or several admission areas (11), which is of limited extent, of the absorbent structure (4); and
- a detection system (10) that is sensitive to the extent of the absorbent structure dampened by the perspiration that has penetrated into it through the admission area or areas (11),
this device comprising, between the application face (2) on the skin and the absorbent structure (4), a structure draining the perspiration that has been perspired by the skin, which is formed completely or partly of a draining material with an absorption capacity that is strictly less than 500 g/mz.

2. Device according to claim 1, the intermediate structure (6) comprising a film (12) forming a barrier to the perspiration, which is crossed by one or several openings (13) connected to the admission area or areas (11), particularly between 1 and 10 openings.

3. Device according to claim 1 or 2, the admission area or areas (11) corresponding to at least 20% of the total surface of the absorbent structure (4), particularly less than 5% of the total surface of the absorbent structure.

4. Device according to claim 2 or 3, the film (12) forming a barrier being hydrophobic.

5. Device according to any one of claims 2 to 4, the film (12) forming a barrier being made of a semi-permeable material, polyurethane for example.

6. Device according to any one of the previous claims, the detection system (10) comprising one or several networks of electrodes, particularly two networks of electrodes (a1,,...an; b1,.... bn) arranged in the form of a grille.

7. Device according to any one of the previous claims, the detection system (10) comprising several measurement locations distributed over the absorbent structure, preferably between 2 and 100 measurement locations, particularly 10 to 50 measurement locations.

8. Device according to any one of the previous claims, the detection system (10) covering a detection surface which corresponds to at least 50% of the total surface of the absorbent structure.

9. Device according to any one of the previous claims, the absorbent structure (4) being formed completely or partly of an absorbent material, having a capacity that is greater than or equal to 800 g/m2.

10. Device according to any one of the previous claims, the absorbent structure (4) being formed completely or partly of a foam material, particularly based on polyurethane foam with open cells, superabsorbent polymer foam, particularly polyacrylates, or fibrous material, particularly based on cellulose fibres.

11. Device according to any one of the previous claims, the structure draining the perspiration being formed completely or partly of a draining material, having an absorption capacity that is between 30 and 500 g/m3.

12. Device according to any one of the previous claims, the draining structure comprising two superimposed layers, the proximal layer (15) on the skin side draining the perspiration axially and the distal layer (16) that is superimposed on it draining the perspiration transversely.

13. Device according to the previous claim, the proximal layer (15) being formed completely or partly of a foam material, particularly polyurethane foam with open cells and/or the distal draining layer (16) being formed completely or partly of a non-woven or knitted material, particularly viscose.

14. Use of a device (1) such as defined according to any one of claims 1 to 13 for assessing the water loss of a person or of an animal through perspiration.

15. Method for assessing the water loss of a person or of an animal through perspiration, in which:
(i) a device according to any one of claims 1 to 13 is applied to a localised area of the skin of the person or of the animal;
(ii) after a certain time, the extent of the absorbent structure dampened by perspiration is detected thanks to the detection system; and
(iii) the amount of perspiration received by the device being worn is determined according to this extent.

16. Method according to the previous claim, in which the detection system (10) is such as defined according to claim 6 and stage (ii) comprises the measurement of an electrical parameter representing the electrical impedance between two electrodes of the detection system using a measuring system (30), in particular stage (ii) being repeated to measure this electrical parameter between different pairs of successive electrodes of the or each network of electrodes.
